(19) **Europäisches Patentamt European Patent Office Office européen des brevets**

(11) **EP 2 145 613 A1**

(12) # EUROPÄISCHE PATENTANMELDUNG

(43) Veröffentlichungstag:
20.01.2010 Patentblatt 2010/03

(51) Int Cl.:
*A61K 6/08* (2006.01)     *A61K 6/083* (2006.01)

(21) Anmeldenummer: 08011903.5

(22) Anmeldetag: 02.07.2008

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Ernst Mühlbauer GmbH & Co.KG 25870 Norderfriedrichskoog (DE)**

(72) Erfinder:
• **Neffgen, Stephan**
 **22459 Hamburg (DE)**
• **Neander, Swen**
 **20148 Hamburg (DE)**

(74) Vertreter: **Glawe, Delfs, Moll Patent- und Rechtsanwälte Rothenbaumchaussee 58 20148 Hamburg (DE)**

(54) **Infiltrant für die Dentalapplikation**

(57) Gegenstand der Erfindung ist ein Infiltrant für die Dentalapplikation, der vernetzende Monomere enthält. Erfindungsgemäß ist vorgesehen, dass er einen Penetrationskoeffizienten PK > 50 cm/s aufweist und die vernetzenden Monomere, bezogen auf die Gesamtmasse der Monomere, wenigstens 5 Gew.-% vernetzende Monomere mit mindestens drei polymerisierbaren Gruppen und höchstens 95 Gew.-% vernetzende Monomere mit zwei polymerisierbaren Gruppen enthalten.

EP 2 145 613 A1

**Beschreibung**

Die Erfindung betrifft einen Infiltranten für die Dentalapplikation, der vernetzende Monomere enthält sowie dessen Verwendung zur Behandlung bzw. Prävention von kariösen Schmelzläsionen.

Unter kariösen Schmelzläsionen sind hier im Wesentlichen sich im Zahnschmelz ausdehnende kariöse Schädigungen zu verstehen, die noch nicht zur Kavitation (Lochbildung) geführt haben. Kariöse Schmelzläsionen sind demineralisierte Bereiche des Zahnschmelzes, die eine Tiefe bis zu etwa 2-3 mm haben können.

Aus der veröffentlichten Internationalen Anmeldung WO 2007/131725 A1 sind zur Behandlung kariöser Schmelzläsionen ein Infiltrationsverfahren und Infiltranten bekannt, so dass die Kavitation verhindert wird und sich die sonst üblicherweise erfolgende Restauration mit Dentalkompositen erübrigt. Bei dem Infiltrationsverfahren wird, nachdem eine evtl. vorhandene remineralisierte oberfläche Schicht entfernt wurde, die Läsion mit einem Infiltranten, der im Wesentlichen aus Monomeren besteht, in Kontakt gebracht, woraufhin diese infiltrieren. Nachdem der Infiltrant die Läsion durchdrungen hat, werden die Monomere mittels Lichtaktivierung polymerisiert. Die Läsion ist dann versiegelt. Das Fortschreiten der Karies wird gestoppt.

Für die Infiltration sind spezielle Monomere bzw. Monomermischungen erforderlich, da bekannte Dentalkleber für Dentalkomposite (auch Adhäsive oder Bondings genannt) zu langsam und/oder nicht ausreichend in die Läsion eindringen und/oder die Läsion vollständig durchdringen (penetrieren oder infiltrieren). Die WO 2007/131725 A1 beschreibt die Verwendung Monomerer bzw. Monomermischungen, so dass der Infiltrant einen Penetrationskoeffizienten PK > 50 hat.

Nachteilig an den dort beschrieben Infiltranten sind deren Erhärtungseigenschaften; insbesondere die Durchhärtungstiefe, sowie die mechanischen Eigenschaften des polymerisierten Infiltranten, insbesondere die Spannungsrissstabilität.

Der Erfindung liegt die Aufgabe zugrunde, einen Infiltranten der eingangs genannten Art zu schaffen, der verbesserte Erhärtungseigenschaften und eine gute Versiegelungswirkung aufweist sowie langlebig ist.

Der erfindungsgemäße Infiltrant weist einen Penetrationskoeffizienten PK > 50 cm/s auf und die vernetzenden Monomere enthalten, bezogen auf die Gesamtmasse aller Monomere des Infiltranten (etwaige Lösemittelanteile fließen in die Berechnung dieser Gesamtmasse nicht ein), wenigstens 5 Gew.-% vernetzende Monomere mit mindestens drei polymerisierbaren Gruppen und höchstens 95 Gew.-% vernetzende Monomere mit zwei polymerisierbaren Gruppen.

Zunächst seien einige im Rahmen der Erfindung verwendete Begriffe erläutert. Der Begriff Infiltrant bezeichnet eine Flüssigkeit, die als ungehärtetes Harz in eine Zahnschmelzläsion (einen porösen Festkörper) eindringen kann. Nach dem Eindringen kann der Infiltrant dort ausgehärtet werden.

Vernetzende Monomere weisen zwei oder mehr polymerisierbare Gruppen auf und können daher bei einer Polymerisation polymerisierte Ketten miteinander vernetzen.

Das Eindringen einer Flüssigkeit (ungehärtetes Harz) in einen porösen Festkörper (Zahnschmelzläsion) wird durch die Washburn-Gleichung (Gleichung 1, siehe unten) physikalisch beschrieben. Bei dieser Gleichung wird angenommen, dass der poröse Festkörper ein Bündel offener Kapillaren darstellt (Buckton G., Interfacial phenomena in drug delivery and targeting. Chur, 1995); in diesem Fall wird das Eindringen der Flüssigkeit von Kapillarkräften getrieben.

$$d^2 = \left( \frac{\gamma \cdot \cos\theta}{2\eta} \right) r \cdot t \qquad - \text{Gleichung 1} -$$

d    Abstand, um den sich das flüssige Harz bewegt
$\gamma$    Oberflächenspannung des flüssigen Harzes (gegen Luft)
$\theta$    Kontaktwinkel des flüssigen Harzes (gegen Zahnschmelz)
$\eta$    dynamische Viskosität des flüssigen Harzes
r    Kapillarradius (Porenradius)
t    Eindringdauer

Der in Klammern stehende Ausdruck der Washburn-Gleichung wird als Penetrationskoeffizient (PK, Gleichung 2, siehe unten) bezeichnet (Fan P. L. et al., Penetrativity of sealants. J. Dent. Res., 1975, 54: 262-264). Der PK setzt sich aus der Oberflächenspannung der Flüssigkeit gegen Luft ($\gamma$), dem Cosinus des Kontaktwinkels der Flüssigkeit gegen Zahnschmelz ($\theta$) und der dynamischen Viskosität der Flüssigkeit ($\eta$) zusammen. Je größer der Wert des Koeffizienten ist, umso schneller dringt die Flüssigkeit in eine gegebene Kapillare oder ein gegebenes poröses Bett ein. Dies bedeutet, dass ein hoher Wert des PK durch hohe Oberflächenspannungen, niedrige Viskositäten und niedrige Kontaktwinkel erzielt werden kann, wobei der Einfluss des Kontaktwinkels vergleichsweise gering ist.

$$PK = \left(\frac{\gamma \cdot \cos\theta}{2\eta}\right) \quad \text{- Gleichung 2 -}$$

PK     Penetrationskoeffizient
$\gamma$     Oberflächenspannung des flüssigen Harzes (gegen Luft)
$\theta$     Kontaktwinkel des flüssigen Harzes (gegen Zahnschmelz)
$\eta$     dynamische Viskosität des flüssigen Harzes

Infiltranten werden häufig in schwer zugänglichen Zahnzwischenräumen (approximal) angewendet. Insbesondere bei der Verwendung lichthärtender Systeme ist eine optimale Bestrahlung zur Durchführung der Aushärtung in Zahnzwischenräumen schwierig. Die Erfindung hat erkannt, dass sich durch die Verwendung einer in Anspruch 1 definierten Mindestmenge vernetzender Monomere mit mindestens drei polymerisierbaren Gruppen eine hinreichende Durchhärttiefe auch unter den genannten schwierigen Bedingungen erreichen lässt und andererseits ein hinreichend hoher Penetrationskoeffizient erhalten bleiben kann, der eine ausreichende Eindringtiefe des Harzes sicherstellt. Die erfindungsgemäße Zusammensetzung des Infiltranten bewirkt somit eine gute Eindringfähigkeit kombiniert mit einer guten Aushärtbarkeit auch unter den schwierigen Bedingungen einer approximalen Applikation.

Bevorzugt liegt der Penetrationskoeffizient des Infiltranten über 100 cm/s, er kann aber auch über 150 oder über 200 cm/s liegen.

Der Anteil vernetzender Monomere mit mindestens drei polymerisierbaren Gruppen kann vorzugsweise zwischen 10 und 50 Gew.-%, weiter vorzugsweise 10 und 30 Gew.-% liegen. Der Anteil vernetzender Monomere mit zwei polymerisierbaren Gruppen liegt bei einer bevorzugten Ausführungsform zwischen 90 und 50 Gew.-%. Ein weiterer bevorzugter Bereich liegt zwischen 90 und 70 Gew.-%.

Die erfindungsgemäßen Infiltranten lassen sich je nach chemischem Aufbau der enthaltenen Monomere radikalisch, anionisch oder kationisch erhärten. Bevorzugt sind die Monomere radikalisch oder kationisch härtbar.

Die radikalische Härtung der erfindungsgemäßen Monomere kann durch Vinylpolymerisation geeigneter Doppelbindungen erfolgen. Besonders geeignet sind hier (Meth)acrylate, (Meth)acrylamide, Styrylverbindungen, Cyanacrylate und Verbindungen mit ähnlich gut radikalisch polymerisierbaren Doppelbindungen. Eine weitere Möglichkeit der radikalischen Härtung besteht in der ringöffnenden Polymerisation von cyclischen Vinylverbindungen wie den in EP1413569, EP1741419 und EP1688125 beschriebenen Vinylcyclopropanen oder anderen cyclischen Systemen wie Vinyliden-substituierten Orthospirocarbonaten oder Orthospiroestern. Eine weitere Möglichkeit besteht auch in der Copolymerisation der ringöffnend polymerisierenden Systeme mit den oben genannten einfach polymerisierenden Doppelbindungen.

Die radikalische Härtung kann darüber hinaus durch eine unter dem Begriff Thiol-En-Reaktion bekannte Stufenreaktionen, wie in WO 2005/086911 beschrieben, erfolgen.
Die kationische Härtung der erfindungsgemäßen Monomere kann ebenfalls durch ringöffnende wie auch durch Vinylpolymerisation erfolgen. Geeignete Vinylpolymere sind Vinylether, Styrylverbindungen und weitere Verbindungen mit elektronenreichen Vinylgruppen. Geeignete ringöffnend polymerisierende Monomere sind Verbindungen, die Epoxid-, Oxetan-, Aziridin-, Oxazolin oder Dioxolangruppen tragen. Weitere ringöffnend polymerisierende Gruppen können der Literatur; bspw.: K.J. Ivin, T. Saegusa, (Eds.), Vol2, Elsevier Appl. Sci. Publ., London 1984; entnommen werden. Besonders geeignet sind siliziumhaltige Epoxidmonomere, wie sie in WO 02/066535 oder WO 2005/121200 beschrieben sind. Besonders vorteilhaft bei der Verwendung von Epoxiden oder Oxetanen ist der geringe Polymerisationsschrumpf wie auch die geringe Inhibitionsschicht dieser Materialien.

Die vernetzenden Monomere mit zwei polymerisierbaren Gruppen sind bevorzugt Ester der Acryl- und/oder Methacrylsäure. Sie können vorzugsweise ausgewählt sein aus der Gruppe bestehend aus DDDMA, 1,10-Decandioldimethacrylat; PEG400DA, Polyethylenglycol 400 Diacrylat; PEG400DMA, Polyethylenglycol 400 Dimethacrylat; PEG300DA, Polyethylenglycol 300 Diacrylat; PEG300DMA, Polyethylenglycol 300 Dimethacrylat; BPA(EO)10DMA, Ethoxyliertes (10) Bisphenol-A-Dimethacrylat; BPA(EO)30DMA, Ethoxyliertes (30) Bisphenol-A-Dimethacrylat; PEG200DA, Polyethylenglycol 200 Diacrylat; PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; BPA(PO)2DMA, Propoxyliertes (2) Bisphenol-A-Dimethacrylat; Bis-GMA, 2,2-bis[4-(2-Hydroxy-3-Methacryloxypropoxy)phenyl]propan; UDMA, 1,6-bis (Methacryloxy-2-Ethoxycarbonylamino)-2,4,4-trimethylhexan; EGDMA, Ethylenglycoldimethacrylat; TEDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; 1,4-Butylendioldiacrylat; 4EDA, Tetraethylenglycoldiacrylat; NDDA, 1,9- Nonandioldiacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat; und Dimethylol tricyclo[5.2.1.0]decandimethacrylat.

Bevorzugte vernetzende Monomere mit mindestens drei polymerisierbaren Gruppen weisen die nachfolgende Formel

$$R^1[X_k\,R^2_1\,Y_m]_n$$

auf, mit folgenden Bedeutungen

$R^1$ ist ein linearer oder verzweigter Kohlenwasserstoff mit 3-24 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether-oder Polyethergruppen, Polyestergruppen, Polysiloxangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;

$R^2$ ist ein linearer oder verzweigter Kohlenwasserstoff mit 1-16 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether-oder Polyethergruppen, Polyestergruppen, Polysiloxangruppen oder Polycarbosilangruppen;

optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;

X ist eine verknüpfende Gruppe gleich oder verschieden ausgewählt aus einer Ethergruppe, Carbonylgruppe, Estergruppe, Amidgruppe, Urethangruppe oder Harnstoffgruppe;

Y ist eine Gruppe gleich oder verschieden enthaltend eine polymerisierbare Doppelbindung und/oder eine ringöffnend polymerisierbare Gruppe und/oder eine Thiolgruppe; bevorzugt Vinyl, (Meth)acrylat, (Meth)acrylamid oder Epoxidgruppen;

k ist 0 oder 1;

l ist 0 oder 1

m ist mindestens 1;

n ist mindestens 1

$m \times n$ ist mindestens 3.

Es kann von Vorteil sein, wenn die Monomeren zusätzliche funktionelle Gruppen aufweisen wie Ammoniumalkylengruppen oder Halogen, insbesondere fluoriertes Alkylen.

Geeignete niedrigviskose Monomeren mit mindestens drei polymerisierbaren Gruppen sind beispielsweise TMPTMA, Trimethylolpropantrimethacrylat, TMPTA, Trimethylolpropantri(meth)acrylat; DTMPTA; Di-Trimethylolpropantetra-(meth)acrylat; DiPENTA, Di-Pentaerythritolpenta(meth)-acrylat; oder DPEHA, Di-Pentaerythritolhexa(meth) acrylat.

Bevorzugte niedrigviskose Monomere mit mindestens drei polymerisierbaren Gruppen basieren bspw. auf alkoxylierten Mehrfachalkoholen (Tri, Tetra-, Penta, Hexa-, Polyole)wie Trimethylolpropan, Ditrimethylolpropan, Glycerol, Pentaerythritol oder Dipentaerythritol.

Besonders bevorzugt sind (Meth)acrylester von alkoxylierten Mehrfachalkoholen wie beispielsweise ethoxyliertes Trimethylolpropan-trimethacrylat, ethoxyliertes Trimethylolpropan-triacrylat, propoxyliertes Trimethylolpropantrimethacrylat, propoxyliertes Trimethylolpropantriacrylat, ethoxyliertes Pentaerythritol-trimethacrylat, ethoxyliertes Pentaerythritol-triacrylat, ethoxyliertes Pentaerythritol-tetramethacrylat, ethoxyliertes Pentaerythritol-tetraacrylat, ethoxyliertes Di-Pentaerythritol-trimethacrylat, ethoxyliertes Di-Pentaerythritol-tetramethacrylat, ethoxyliertes Di-Pentaerythritolpentamethacrylat, ethoxyliertes Di-Pentaerythritolhexamethacrylat, ethoxyliertes Di-Pentaerythritol-triacrylat, ethoxyliertes Di-Pentaerythritol-tetraacrylat, ethoxyliertes Di-Pentaerythritol-pentaacrylat, ethoxyliertes Di-Pentaerythritol-hexaacrylat, propoxyliertes Pentaerythritol-trimethacrylat, propoxyliertes Pentaerythritol-triacrylat, propoxyliertes Pentaerythritol-tetramethacrylat, propoxyliertes Pentaerythritol-tetraacrylat, propxyliertes Di-Pentaerythritol-trimethacrylat, propoxyliertes Di-Pentaerythritol-tetramethacrylat, propoxyliertes Di-Pentaerythritolpentamethacrylat, propoxyliertes Di-Pentaerythritolhexamethacrylat, propoxyliertes Di-Pentaerythritol-triacrylat, propoxyliertes Di-Pentaerythritol-tetraacrylat, propxyliertes Di-Pentaerythritol-pentaacrylat, propoxyliertes Di-Pentaerythritol-hexaacrylat.

Solche an die Alkohole angebunden Alkoxygruppen stellen (Molekül)Kettenverlängerer dar. Die Kettenverlängerung kann vorzugsweise durch Ethoxylierung oder Propoxylierung erreicht werden. Für die Kettenverlängerung kommen weitere Verknüpfungsmöglichkeiten bspw. Etherbindungen, Esterbindungen, Amidbindungen, Urethanbindungen u.ä. in Frage, an die sich bevorzugt wiederum Ethylenglykol- oder Propylenglykolgruppen anschließen können.

Bevorzugte Kettenverlängerer sind bspw.

$-CH_2-CH_2-$

$-CH_2-CH_2-CH_2-$

$-CH_2-CH_2-O-CH_2-CH_2-$

$-CH_2-CH_2-CH_2-O-CH_2-CH_2-CH_2-$

u.s.w.

$-O-CH_2-CH_2-$

$-O-CH_2-CH_2-CH_2-$

$-O-CH_2-CH_2-O-CH_2-CH_2-$

$-O-CH_2-CH_2-CH_2-O-CH_2-CH_2-CH_2-$

u.s.w.

$-O-CO-CH_2-CH_2-$

$-O-CO-CH_2-CH_2-CH_2-$

$-O-CO-CH_2-CH_2-O-CH_2-CH_2-$

$-O-CO-CH_2-CH_2-CH_2-O-CH_2-CH_2-CH_2-$

u.s.w.

$-CO-CH_2-CH_2-$

$-CO-CH_2-CH_2-CH_2-$

$-CO-CH_2-CH_2-O-CH_2-CH_2-$

$-CO-CH_2-CH_2-CH_2-O-CH_2-CH_2-CH_2-$

u.s.w.

$-NR^3-CO-CH_2-CH_2-$

$-NR^3-CO-CH_2-CH_2-CH_2-$

$-NR^3-CO-CH_2-CH_2-O-CH_2-CH_2-$

$-NR^3-CO-CH_2-CH_2-CH_2-O-CH_2-CH_2-CH_2-$

u.s.w.

$-O-CO-NR^3-CH_2-CH_2-$

$-O-CO-NR^3-CH_2-CH_2-CH_2-$

$-O-CO-NR^3-CH_2-CH_2-O-CH_2-CH_2-$

$-O-CO-NR^3-CH_2-CH_2-CH_2-O-CH_2-CH_2-CH_2-$

u.s.w.

$-NR^3-CO-O-CH_2-CH_2-$

$-NR^3-CO-O-CH_2-CH_2-CH_2-$

$-NR^3-CO-O-CH_2-CH_2-O-CH_2-CH_2-$

$-NR^3-CO-O-CH_2-CH_2-CH_2-O-CH_2-CH_2-CH_2-$

u.s.w.

$-NR^3-CO-NR^3-CH_2-CH_2-$

$-NR^3-CO-NR^3-CH_2-CH_2-CH_2-$

$-NR^3-CO-NR^3-CH_2-CH_2-O-CH_2-CH_2-$

$-NR^3-CO-NR^3-CH_2-CH_2-CH_2-O-CH_2-CH_2-CH_2-$

u.s.w.

wobei $R^3$ bevorzugt H oder eine Methylgruppe ist.

Die kettenverlängernde Gruppe ist bevorzugt endständig mit den vernetzenden Gruppen, bevorzugt einer Methacrylat, einer Acrylatgruppe, Methacrylamid oder Acrylamidgruppe funktionalisiert.

Als Vernetzungspunkt wird die Position der vernetzenden polymerisierbaren Gruppe bspw. die Position einer C=C-Doppelbindung im Monomer angesehen.

Die Kettenlänge ist bevorzugt derart, dass der Abstand der Vernetzungspunkte mindestens 7, bevorzugt mindestens 9, weiter bevorzugt 10 bis 30, besonders bevorzugt 11 bis 21 Bindungslängen beträgt. Der Abstand beträgt bevorzugt weniger als 50 Bindungslängen.

Mit Abstand der Vernetzungspunkte, ist der kürzeste Abstand der vernetzenden Gruppen bspw. zweier C=C-Doppelbindungen entlang des Moleküls zu verstehen. Es ist also nur die Konstitution des Moleküls gemeint, nicht etwa die wirkliche räumliche Lage der Gruppen zueinander, etwa durch Konfiguration oder Konformation bedingt.

Unter Bindungslänge ist der Abstand zweier Atome im Molekül zu verstehen, egal welche Art kovalente Bindung vorliegt und welche exakte Bindungslänge die einzelne kovalente Bindung aufweist.

Der Anteil der vernetzenden Monomere mit mindestens drei polymerisierbaren Gruppen und einem Abstand der Vernetzungspunkte von weniger als 10 Bindungslängen, bezogen auf die Gesamtmasse der Monomere, beträgt vorzugsweise weniger als 20 Gew.-%, weiter vorzugsweise weniger als 10 Gew.-%, weiter vorzugsweise weniger als 5 Gew.-%.

Die Erfindung hat erkannt, dass durch die Einstellung eines Abstandes zwischen den vernetzenden Gruppen, wie oben definiert, und damit einhergehend die Einstellung einer entsprechenden Netzbogenlänge in vernetzten Polymeren die Spannungen im ausgehärteten Polymer vermindert werden. Diese Verminderung der Spannungen führt dazu, dass es auch unter Temperaturwechselbelastungen wie beispielsweise einem als Testverfahren verwendeten Thermocycling zwischen 5 und 55°C nicht zu spannungsinduzierten Rissbildungen im Polymer kommt. Die mechanischen Eigenschaften und insbesondere die Dauerbeständigkeit des ausgehärteten Infiltranten werden so verbessert.

Der bevorzugte Anteil dieser Monomeren hängt von der Anzahl der vernetzenden Gruppen in der Monomermischung, der Größe der kettenverlängernden Gruppen und dem resultierenden PK ab.

Der erfindungsgemäße Infiltrant kann zusätzlich Monomere mit einer polymerisierbaren Gruppe enthalten. Diese können bevorzugt ausgewählt sein aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidyl-methacrylat; THFMA, Tetrahydrofurfurylmethacrylat; AMA, Allylmethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; ; EHA, 2-Ethylhexylacrylat; und 3EGMA, Triethylenglycolmonomethacrylat.

Die Monomere, Monomermischungen und/oder Infiltranten haben bevorzugt eine Dynamische Viskosität kleiner 50 mPas, weiter bevorzugt kleiner 30 mPas, besonders bevorzugt kleiner 15 mPas.

Die Mischung unterschiedlicher Monomeren dient insbesondere der Abstimmung der mechanischen Eigenschaften wie Härte und Festigkeit, der Durchhärtetiefe bzw. des Polymerisationsgrades, des Restmonomergehaltes, der Schmierschichtausprägung, des Schrumpfs, der Stabilität, der Wasseraufnahme sowie insbesondere der Spannungsfreiheit unter Erhalt einer hohen Penetrationsfähigkeit (PK > 50).

Insbesondere ist auch die Wasserverträglichkeit der Monomeren von Bedeutung etwa für den Fall, dass die Schmelzläsion nach der Vorbereitung (Ätzen, Spülen, Trocknen) noch Restfeuchtigkeit aufweist. Bestimmte Monomere können Restfeuchtigkeit aufnehmen und so die Penetration weiter verbessern. Hierfür eignen sich besonders wasserlösliche und/oder phasenvermittelnde Ester der (Meth)acrylsäure, bspw. HEMA, 2-Hydroxyethylmethacrylat oder GDMA, Glycerindimethacrylat oder GMA, Glycerinmonomethacrylat.

Die Mischung mit weiteren Monomeren kann weiterhin insbesondere der Abstimmung weiterer vorteilhafter Eigenschaften wie hohe Oberflächenglätte (plaqueverhindernd), Fluoridfreisetzung, Röntgenopazität, Haftung am Schmelz, Langzeitfarbstabilität, Biokompatibilität u.s.w. dienen.

Der Infiltrant kann auch dem Fachmann bspw. aus WO 02/062901, WO 2006/031972 oder EP 1714633 bekannte und im Dentalbereich gebräuchliche hyperverzweigte Monomere, bspw. Dendrimere aufweisen, insbesondere um den Restmonomergehalt zu senken und die Biokompatibilität zu verbessern.

Der Infiltrant kann dem Fachmann bspw. aus EP 1285947 oder EP 1849450 bekannte und im Dentalbereich gebräuchliche bakterizide Monomere enthalten.

Die Monomermischungen bzw. der Infiltrant haben dabei einen PK > 50, bevorzugt > 100, besonders bevorzugt > 200.

Der Infiltrant enthält Mittel zur Härtung des Infiltranten. Die Mittel zur Härtung können dem Fachmann bekannte und im Dentalbereich gebräuchliche Initiatoren insbesondere lichtaktivierte Initiatorsysteme aber auch chemisch aktivierende Initiatoren sein oder Mischungen der verschiedenen Systeme.

Die hier verwendbaren Initiatoren können z. B. Photoinitiatoren sein. Diese sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm und optional durch die zusätzliche Reaktion mit einem oder mehreren Coinitiatoren die Aushärtung des Materials bewirken können. Bevorzugt werden hier Phosphinoxide, Bezoinether, Benzilketale, Acetophenone, Benzophenone, Thioxanthone, Bisimidazole, Metallocene, Fluorone, $\alpha$-Dicarbonylverbindungen, Aryldiazoniumsalze, Arylsulfoniumsalze, Aryliodoniumsalze, Ferroceniumsalze, Phenylphosphoniumsalze oder eine Mischung aus diesen Verbindungen eingesetzt.

Besonders bevorzugt werden Diphenyl-2,4,6-trimethylbenzoyl-phosphinoxid, Benzoin, Benzoinalkylether, Benzildialkylketale, α-Hydroxy-acetophenon, Dialkoxyacetophenone, α-Aminoacetophenone, i-Propylthioxanthon, Campherchinon, Phenylpropandion, 5,7-Diiodo-3-butoxy-6-fluoron, (eta-6-Cumol)(eta-5-cyclopentadienyl)eisen-hexafluorophosphat, (e-ta-6-Cumol)(eta-5-cyclopentadienyl)eisen-tetrafluoroborat, (eta-6-Cumol)(eta-5-cyclopentadienyl)eisen-hexafluoroantimonat, substituierte Diaryliodoniumsalze, Triarylsulfoniumsalze oder eine Mischung aus diesen Verbindungen eingesetzt.

Als Coinitiatoren für eine photochemische Aushärtung werden bevorzugt tertiäre Amine, Borate, organische Phosphite, Diaryliodoniumverbindungen, Thioxanthone, Xanthene, Fluorene, Fluorone, α-Dicarbonylverbindungen, kondensierte Polyaromaten oder eine Mischung aus diesen Verbindungen eingesetzt. Besonders bevorzugt werden N,N-Dimethyl-p-toluolidin, N,N-Dialkyl-alkyl-aniline, N,N-Dihydroxyethyl-p-toluidin, 2-Ethylhexyl-p-(dimethyl-amino)-benzoat, Butyrylcholin-triphenylbutyl-borat oder eine Mischung aus diesen Verbindungen eingesetzt.

Der Infiltrant kann aus einem Kit mit wenigstens zwei Komponenten hergestellt werden. Zwei Komponenten enthaltende Infiltranten haben den Vorteil, dass sie selbsthärtend (chemische Aushärtung) gestaltet werden können. In einer Ausführungsform enthält eine erste Komponente Monomere und chemisch aktivierbare Initiatoren und eine zweite Komponente geeignete Aktivatoren.

Für eine chemische Aushärtung bei Raumtemperatur wird i. a. ein Redoxinitiatorsystem verwendet, das aus einem bzw. mehreren Initiatoren und einem als Aktivator dienenden Coinitiator bzw. Coinitiatoren besteht. Aus Gründen der Lagerstabilität werden Initiator bzw. Initiatoren und Coinitiator bzw. Coinitiatoren in räumlich voneinander getrennten Teilen des erfindungsgemäßen Infiltranten eingearbeitet, d. h. es liegt ein mehrkomponentiges, bevorzugt ein zweikomponentiges Material vor. Als Initiator bzw. Initiatoren werden bevorzugt anorganische und/oder organische Peroxide, anorganische und/oder organische Hydroperoxide, Barbitursäurederivate, Malonylsulfamide, Protonensäuren, Lewis- oder Broensted-Säuren bzw. Verbindungen, die solche Säuren freisetzen, Carbeniumionen-Donatoren wie z. B. Methyltriflat oder Triethylperchlorat oder eine Mischung aus diesen Verbindungen und als Coinitiator bzw. als Coinitiatoren bevorzugt tertiäre Amine, Schwermetallverbindungen, insbesondere Verbindungen der 8. und der 9. Gruppe des Periodensystems ("Eisen- und Kupfergruppe"), Verbindungen mit ionogen gebundenen Halogenen oder Pseudohalogenen wie z. B. quartäre Ammoniumhalogenide, schwache Broenstedt-Säuren wie z. B. Alkohole und Wasser oder eine Mischung aus diesen Verbindungen eingesetzt.

In einer besonders einfachen Ausführungsform ist das Gerät zum Auftragen des Infiltranten (Applikationshilfe) auf den Zahn mit dem Aktivator getränkt oder belegt.

In einer weiteren Ausführungsform enthält eine erste Komponente Monomere und als Initiatoren Salze CHazider Verbindungen wie Barbitursäurederivate und eine zweite Komponente Monomere und eine aktivierende Komponente, bevorzugt eine stärker azide Säure als die CH-azide Verbindung.

In einer besonders einfachen Ausführungsform enthält ein Gerät zum Auftragen des Infiltranten (Applikationshilfe) auf den Zahn eine Mischkammer und/oder Mischelemente enthaltende Kanülen.

Der Infiltrant kann Stabilisatoren enthalten. Bevorzugt werden UV-Stabilisatoren. Geeignete UV-Stabilisatoren sind dem Fachmann bekannt beispielhaft sei hier Chimasorb® und Tinuvin® (Ciba) genannt.

Der Infiltrant kann Lösungsmittel enthalten. Bevorzugt sind flüchtige Lösungsmittel wie bspw. Alkohole, Ketone, Ether, u.s.w.

Bevorzugt enthält der Infiltrant weniger als ungefähr 20 Masse-%, weiter bevorzugt weniger als ungefähr 10 Masse-%, besonders bevorzugt kein Lösungsmittel.

Der Infiltrant kann mindestens einen fluoreszierenden Farbstoff und/oder Farbpigmente enthalten, um das Erscheinungsbild zu verbessern bzw. dem Zahnschmelz anzupassen. Geeignete fluoreszierende Farbmittel sind dem Fachmann bekannt und bspw. in der US 2004/017928 A1 beschrieben. Der Infiltrant kann sonstige Farbmittel insbesondere zur Herstellung verschiedener Zahnfarben enthalten. Der Infiltrant kann die Farbe wechselnde Farbstoffe enthalten, die die infiltrierte Läsion anzeigen und durch Farbumschlag die Aushärtung des Infiltranten anzeigen. Bevorzugt wird der Farbstoff nach Aushärtung des Infiltranten farblos. Der Farbstoff kann radikalisch reaktiv sein.

Der Farbumschlag kann auch von anderen Einflüssen abhängen bspw. vom pH-Wert.

Der Farbstoff kann adsorptive Eigenschaften, insbesondere bezüglich des Zahnschmelzes aufweisen, so dass er sich in der oberen Schicht der Läsion anreichert. Der Farbumschlag kann dann auch im Zwischenzahnbereich besser gesehen werden.

Der Farbstoff kann nicht-adsorptive Eigenschaften aufweisen und tief in die Läsion penetrieren, so dass bspw. die Durchdringung besser kontrolliert werden kann.

Der Infiltrant kann thermo- und/oder photochrome Zusätze enthalten, durch die der infiltrierte Bereich bei Bestrahlung mit entsprechendem Licht bzw. Temperaturänderung angezeigt wird.

Gegenstand der Erfindung ist ferner die Verwendung eines erfindungsgemäßen Infiltranten zur Behandlung und/oder Prävention von kariösen Schmelzläsionen. Eine solche Verwendung kann die nachfolgenden Schritte umfassen:

1. Entfernen einer dünnen Oberflächenschicht der Schmelzläsion durch Ätzmittel
2. Abspülen des Ätzmittels
3. Trocknen der Läsion mit einem Trocknungsmittel
4. Infiltration der Läsion mit einem Infiltranten
5. Entfernen von Überschüssen (optional)
6. Härten des Infiltranten
7. Infiltration der Läsion mit einem Infiltrant (optional)
8. Entfernen von Überschüssen (optional)
9. Härten des Infiltranten (optional)
10. Politur der infiltrierten Läsionsoberfläche (optional)

Bei einzelnen Schritten der Infiltrationsmethode kann das gewünschte Ergebnis durch Anwendung von Schall und/oder Ultraschall weiter verbessert werden.

Bevorzugte Ätzmittel sind Gele starker Säuren wie Salzsäure.

Bevorzugte Trocknungsmittel sind toxikologisch unbedenkliche Lösungsmittel mit hohem Dampfdruck. Diese sind bspw. ausgewählt aus Alkoholen, Ketonen, Ethern, Estern usw. Besonders bevorzugt ist Ethanol.

Das Trocknungsmittel kann Bestandteile des Initiatorsystems enthalten, die nach dessen Verdunstung in der Läsion verbleiben.

Das Trocknungsmittel kann einen Filmbildner enthalten.

Die Erfindung umfasst weiterhin einen Kit zur Durchführung des Infiltrationsverfahrens. Dieser umfasst

1. Ätzmittel
2. Trocknungsmittel
3. Infiltrant.

Im Folgenden ist die Erfindung anhand einiger Beispiele erläutert.

Monomermischungen wurden hergestellt und deren Penetrationskoeffizient (PK), Durchhärtetiefe und mechanisches sowie thermomechanisches Verhalten überprüft(Schlagzähigkeit, Thermocycling).

Die folgenden Komponenten kamen zum Einsatz:

| TEDMA | Triethlenglycoldimethacrylat |
|---|---|
| E3TMPTA | Trimethylolpropan ethoxyliert mit durchschn. 1 EO-Einheiten pro Methylolgruppe und terminal acryliert |
| HEMA | 2-Hydroxyethylmethacrylat |
| HDDMA | 1,6-Hexandioldimethacrylat |
| PEA | Phenoxyethylacrylat |
| CQ | Kampherchinon |
| EHA | Ethylhexyl-p-N,N-dimethylaminobenzoat |
| BHT | 2,6-Di-tert.-butylphenol |

Prüfungsmethoden

*Oberflächenspannung*

Die Oberflächenspannung der Harze wurde mittels Konturanalyse eines hängenden Tropfens durchgeführt (DSA 10, KRÜSS GmbH). Die Messung der Oberflächenspannung wurde an neu gebildeten Tropfen über einen Zeitraum von 30 s durchgeführt, wobei etwa alle 5 s ein Messwert aufgenommen wurde. Hierzu wurden die Harze mit einer feinen Spritze ausgebracht und der sich bildende Tropfen mit einer Digitalkamera gefilmt. Aus der charakteristischen Form und Größe des Tropfens wurde die Oberflächenspannung gemäß der Young-Laplace-Gleichung bestimmt. Für jedes Harz wurden so 3 Messungen durchgeführt, deren Mittelwert als Oberflächenspannung angegeben wurde.

*Dichtebestimmung*

Die Dichten der Harze wurden mittels Pyknometer bestimmt. Hierzu wurde die Dichte der Luft mit 0,0013 g/ml und die Erdbeschleunigung mit $9,8100$ m/s$^2$ berücksichtigt.

*Kontaktwinkel*

Für jede Einzelmessung wurde Schmelz aus Rinderzähnen verwendet. Hierzu wurden Rinderzähne in einem Kunstharz eingebettet und die Schmelzfläche mittels Schleifmaschine (Struers GmbH) mit Schleifpapieren (80, 500 und 1200 Körnung) nass poliert, so dass plane etwa 0,5x1,0 cm große Schmelzflächen für die Kontaktwinkel-Messungen zur Verfügung standen. Die Schmelzproben wurden bis zur Messung in destilliertem Wasser gelagert und vor der Messung mit Ethanol und Druckluft getrocknet.

Die Messung des Kontaktwinkels erfolgte mit Hilfe eines Video-Kontaktwinkelmessgerät (DSA, KRÜSS GmbH). Hierbei wurde mittels Mikroliterspritze ein Tropfen der Harzmischung auf die Schmelzfläche gebracht, innerhalb von 10 s bis zu 40 Einzelaufnahmen des Tropfens rechnergesteuert aufgenommen und per Tropfenkonturanalyse-Software der Kontaktwinkel ermittelt.

*Durchhärtetiefe*

Die Durchhärtetiefen der Harze wurden entsprechend der Prüfung "Polymerisationstiefe" nach ISO 4049:2000 bestimmt. Hierzu wurden die Harze in zylindrische Teflonformen (5 mm im Durchmesser, 10 mm hoch) gefüllt und mit einer Halogenlampe (Translux EC, Heraeus Kulzer GmbH) 20, 40 oder 60 s von oben belichtet. Direkt nach der Belichtung wurden die ausgehärteten Probekörper entformt und mit einem Plastikspatel von unausgehärtetem Material befreit. Die Höhe des ausgehärteten Zylinderkegels wurde als Durchhärtetiefe (DHT) angegeben.

*Thermomechanische Prüfung*

Die Überprüfung der thermomechanischen Belastbarkeit der ausgehärteten Harze erfolgte mittels zylindrischer Prüfkörper (25 mm im Durchmesser; 2,5 mm hoch). Die Prüfkörper wurden hergestellt indem die jeweiligen Harzmischungen in entsprechenden Formen 5 min in einem Lichtpolymerisationsgerät (Beleuchtungsstärke 15200 lux) bestrahlt und anschließend 23 h bei 40°C im Trockenschrank gelagert wurden.

Die Prüfkörper wurden einem Temperaturwechsel (Thermocycler, Willytec GmbH) ausgesetzt indem sie abwechselnd in Wasserbäder mit Temperaturen von 55°C und 5°C getaucht wurden. Die Eintauchzeit betrug jeweils 30 s, die Abtropfzeit jeweils 10 s, sodass ein Tauchzyklus 40 s dauerte. Die Prüfkörper wurden jeweils 5000 Tauchzyklen ausgesetzt. Anschließend wurden die Prüfkörper auf Riss- und Mikrorissbildung untersucht.

Schlagzähigkeit

Zunächst wurden hierfür Prüfkörper hergestellt indem die Harze jeweils in Prüfkörperformen (15 x 10 x 3 mm) 360 s bestrahlt (Heraflash Polymerisationslichtgerät, Heraeus Kulzer GmbH) und 24 Stunden bei 37°C in destilliertem Wasser gelagert wurden.

Von den auf 23°C temperierten Prüfkörper wurde die exakten Abmessungen ermittelt und anschließend die Schlagzähigkeit gemäß DIN 53435 über die Schlagarbeit ermittelt (Dynstat Prüfgerät, Karl Frank GmbH). Es wurden 8 Einzelmessungen durchgeführt deren Mittelwert als Schlagzähigkeit angegeben wurde.

*Dynamische Viskosität*

Die Viskosität der Harze wurde bei 23°C mittels dynamischen Platte-Platte-Viskosimeter (Dynamic Stress Rheometer, Rheometric Scientific Inc.) gemessen. Es wurde im "Steady Stress Sweep"-Modus bei Spaltgrößen von 0,1 bis 0,5 mm im Bereich von 0 bis 50 Pa Schubspannung ohne Vorscherung der Harze gemessen.

Beschreibung der Herstellung von Beispielen und Referenzbeispielen

Die Harze wurden gemäß Tabelle durch Verrühren der entsprechenden Komponenten hergestellt. Für die Untersuchungen Schlagzähigkeit, Durchhärtetiefe und thermomechanische Stabilität wurden zu den Harzmischungen 0,5 Gew% CQ, 0,84 Gew% EHA und 0,002 Gew% BHT zugesetzt. Alle Mischungen wurden bis zur optisch klaren Lösung gerührt

EP 2 145 613 A1

| | Harz 1 | Harz 2 | Harz 3 | Harz 4 | Harz 5 | Harz 6 | Harz 7 | Harz 8 | Harz 9 | Harz 10 | Harz 11 |
|---|---|---|---|---|---|---|---|---|---|---|---|
| HEMA | | | | | | | 10 | 20 | 30 | | |
| PEA | | | | | | 20 | | | | | |
| MDP | | | | | | | | | | | |
| HDDMA | | | | | | | | | | 100 | 50 |
| TEDMA | 100 | 80 | 85 | 90 | 95 | 60 | 70 | 60 | 50 | | |
| TMP(EO)$_3$TA | | 20 | 15 | 10 | 5 | 20 | 20 | 20 | 20 | | 50 |
| | | | | | | | | | | | |
| Dichte | 1,075 | 1,084 | | | 1,052 | 1,052 | 1,090 | 1,091 | | 0,997 | 1,044 |
| Viskosität [mPas] | 10 | 12 | | | 10 | 12 | 11,5 | 11 | | 5 | 15 |
| Oberflächensp.[mN/m] | 35,07 | 35,37 | | | 34,22 | 34,66 | 35,52 | 35,49 | | 32,51 | 33,94 |
| Kontaktwinkel Schmelz [°] | 0,4 | 2,4 | | | 1,1 | 1,5 | 1,2 | 1,0 | | 2,0 | 7,5 |
| Penetrationskoeffiz.[cm/s] | 175 | 147 | – | – | 171 | 144 | 154 | 161 | – | 325 | 112 |
| DHT [mm] 20s | 0 | 4,1 | 0 | 0 | 0 | 4,3 | 0 | 0 | 0 | 0 | 7,8 |
| 40s | 0 | 8 | 5,6 | 0 | 0 | – | 6,2 | 2,9 | 2 | – | – |
| 60s | 0 | 10 | 10 | 5,2 | 5,2 | – | – | – | – | – | – |
| Schlagzähigkt | 3,5(0,3) | 6,0(3,2) | – | – | 6,4(2,3) | 7,2(1,6) | 3,1(0,8) | 3,6(1,4) | – | 4,3(0,7) | 4,9(1,0) |
| Rißbildung nach TC | keine | keine | keine | – | keine | keine | keine | keine | keine | ja | keine |

**Patentansprüche**

1. Infiltrant für die Dentalapplikation, der vernetzende Monomere enthält, **dadurch gekennzeichnet, dass** er einen Penetrationskoeffizienten PK > 50 cm/s aufweist und die vernetzenden Monomere, bezogen auf die Gesamtmasse der Monomere, wenigstens 5 Gew.-% vernetzende Monomere mit mindestens drei polymerisierbaren Gruppen und höchstens 95 Gew.-% vernetzende Monomere mit zwei polymerisierbaren Gruppen enthalten.

2. Infiltrant nach Anspruch 1, **dadurch gekennzeichnet, dass** er einen Penetrationskoeffizienten PK > 100 cm/s aufweist.

3. Infiltrant nach Anspruch 1 oder 2, **dadurch gekennzeichnet, dass** der Anteil vernetzender Monomere mit mindestens drei polymerisierbaren Gruppen 10 bis 50 Gew.-%, vorzugsweise 10 bis 30 Gew.-%, beträgt.

4. Infiltrant nach einem der Ansprüche 1 bis 3, **dadurch gekennzeichnet, dass** der Anteil vernetzender Monomere mit zwei polymerisierbaren Gruppen 90 bis 50 Gew.-%, vorzugsweise 90 bis 70 Gew.-%, beträgt.

5. Infiltrant nach einem der Ansprüche 1 bis 4, **dadurch gekennzeichnet, dass** die vernetzenden Monomere mit zwei polymerisierbaren Gruppen Ester der Acryl- oder Methacrylsäure sind und vorzugsweise ausgewählt sind aus der Gruppe bestehend aus DDDMA, 1,10-Decandioldimethacrylat; PEG400DA, Polyethylenglycol 400 Diacrylat; PEG400DMA, Polyethylenglycol 400 Dimethacrylat; PEG300DA, Polyethylenglycol 300 Diacrylat; PEG300DMA, Polyethylenglycol 300 Dimethacrylat; BPA(EO)10DMA, Ethoxyliertes (10) Bisphenol-A-Dimethacrylat; BPA(EO) 30DMA, Ethoxyliertes (30) Bisphenol-A-Dimethacrylat; PEG200DA, Polyethylenglycol 200 Diacrylat; PEG600DA, Polyethylenglycol 600 Diacrylat; NPG(PO)2DA Propoxyliertes (2) Neopentylglycol Diacrylat; BPA(EO)2DA, Ethoxyliertes (4) Bisphenol-A-Diacrylat; BPA(PO)2DMA, Propoxyliertes (2) Bisphenol-A-Dimethacrylat; Bis-GMA, 2,2-bis [4-(2-Hydroxy-3-Methacryloxypropoxy)phenyl]propan; UDMA, 1,6-bis(Methacryloxy-2-Ethoxycarbonylamino)- 2,4,4-trimethylhexan; EGDMA, Ethylenglycoldimethacrylat; 3EGDMA, Triethylenglycoldimethacrylat; 4EGDMA, Tetraethylenglycoldimethacrylat; BDMA, 1,3-Butylenglycoldimethacrylat; HDDMA, 1,6-Hexandioldimethacrylat; 1,4- Butylendioldiacrylat; 4EDA, Tetraethylenglycoldiacrylat; NDDA, 1,9- Nonandioldiacrylat; DEGDMA, Diethylenglycoldimethacrylat; PDDMA, 1,5-Pentandioldimethacrylat; BDDMA, 1,4-Butandioldimethacrylat; PRDMA, 1,3-Propandioldimethacrylat; und DMTCDDA, Dimethylol tricyclo[5.2.1.0]decandimethacrylat.

6. Infiltrant nach einem der Ansprüche 1 bis 5, **dadurch gekennzeichnet, dass** die vernetzenden Monomere mit mindestens drei polymerisierbaren Gruppen einen Abstand der Vernetzungspunkte von wenigstens 7 Bindungslängen, vorzugsweise höchstens 50 Bindungslängen, weiter vorzugsweise 10 bis 30 Bindungslängen, weiter vorzugsweise 11 bis 21 Bindungslängen aufweisen.

7. Infiltrant nach einem der Ansprüche 1 bis 6, **dadurch gekennzeichnet, dass** der Anteil der vernetzenden Monomere mit mindestens drei polymerisierbaren Gruppen und einem Abstand der Vernetzungspunkte von weniger als 10 Bindungslängen, bezogen auf die Gesamtmasse der Monomere, weniger als 20 Gew.-%, vorzugsweise weniger als 10 Gew.-%, weiter vorzugsweise weniger als 5 Gew.-% beträgt.

8. Infiltrant nach einem der Ansprüche 1 bis 7, **dadurch gekennzeichnet, dass** die vernetzenden Monomere mit mindestens drei polymerisierbaren Gruppen die nachfolgende Formel aufweisen,

$$R^1[X_k\, R^2_1\, Y_m]_n$$

mit folgenden Bedeutungen
$R^1$ ist ein linearer oder verzweigter Kohlenwasserstoff mit 3-24 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen;
optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;
$R^2$ ist ein linearer oder verzweigter Kohlenwasserstoff mit 1-16 C-Atomen, enthaltend Alkyl, Cycloalkyl oder Aryl; optional enthaltend O, N, Si, S, P als Heteroatome, beispielsweise Siloxan und/oder Cyclosiloxan und/oder Carbosilan und/oder Cyclocarbosilan und/oder insbesondere Ether- oder Polyethergruppen, Polyestergruppen, Polysiloxangruppen oder Polycarbosilangruppen;
optional substituiert durch Hydroxyl und/oder Carbonyl und/oder Halogen (bevorzugt Fluor) und/oder Ammoniumalkylengruppen und/oder Siloxan und/oder Cyclosiloxan und/oder Carbosilan und/oder Cyclocarbosilan;

X ist eine verknüpfende Gruppe gleich oder verschieden ausgewählt aus einer Ethergruppe, Carbonylgruppe, Estergruppe, Amidgruppe, Urethangruppe oder Harnstoffgruppe;

Y ist eine Gruppe gleich oder verschieden enthaltend eine polymerisierbare Doppelbindung und/oder eine ringöffnend polymerisierbare Gruppe und/oder eine Thiolgruppe; bevorzugt Vinyl, (Meth)acrylat, (Meth)acrylamid oder Epoxidgruppen;

k ist 0 oder 1;

l ist 0 oder 1

m ist mindestens 1

n ist mindestens 1

$m \times n$ ist mindestens 3.

9. Infiltrant nach einem der Ansprüche 1 bis 8, **dadurch gekennzeichnet, dass** die vernetzenden Monomere mit mindestens drei polymerisierbaren Gruppen ausgewählt sind aus der Gruppe bestehend aus TMPTMA, Trimethylolpropantrimethacrylat; TMPTA, Trimethylolpropantriacrylat; DTMPTA, Di-Trimethylolpropantetra(meth)acrylat; Di-PENTA, Di-Pentaerythritolpenta(meth)acrylat; GPTA, propoxyliertes Glyceryltri(meth)acrylat; DPEHA, Dipentaerythritolhexa(meth)acrylat; und ethoxyliertes Trimethylolpropantri(meth)acrylat; vorzugsweise ausgewählt sind aus der Gruppe bestehend aus propoxyliertes Glyceryltri(meth)acrylat; ethoxyliertes Trimethylolpropantrimethacrylat, ethoxyliertes Trimethylolpropantriacrylat, propoxyliertes Trimethylolpropantrimethacrylat, propoxyliertes Trimethylolpropantriacrylat, ethoxyliertes Pentaerythritol-trimethacrylat, ethoxyliertes Pentaerythritol-triacrylat, ethoxyliertes Pentaerythritol-tetramethacrylat, ethoxyliertes Pentaerythritol-tetraacrylat, ethoxyliertes Di-Pentaerythritol-trimethacrylat, ethoxyliertes Di-Pentaerythritol-tetramethacrylat, ethoxyliertes Di-Pentaerythritolpentamethacrylat, ethoxyliertes Di-Pentaerythritolhexamethacrylat, ethoxyliertes Di-Pentaerythritol-triacrylat, ethoxyliertes Di-Pentaerythritol-tetraacrylat, ethoxyliertes Di-Pentaerythritol-pentaacrylat, ethoxyliertes Di-Pentaerythritol-hexaacrylat, propoxyliertes Pentaerythritol-trimethacrylat, propoxyliertes Pentaerythritol-triacrylat, propoxyliertes Pentaerythritol-tetramethacrylat, propoxyliertes Pentaerythritol-tetraacrylat, propxyliertes Di-Pentaerythritol-trimethacrylat, propoxyliertes Di-Pentaerythritol-tetramethacrylat, propoxyliertes Di-Pentaerythritolpentamethacrylat, propoxyliertes Di-Pentaerythritolhexamethacrylat, propoxyliertes Di-Pentaerythritol-triacrylat, propoxyliertes Di-Pentaerythritol-tetraacrylat, propxyliertes Di-Pentaerythritol-pentaacrylat, propoxyliertes Di-Pentaerythritol-hexaacrylat.

10. Infiltrant nach einem der Ansprüche 1 bis 9, **dadurch gekennzeichnet, dass** er zusätzlich Monomere mit nur einer polymerisierbaren Gruppe enthält; wobei die Monomere mit nur einer polymerisierbaren Gruppe bevorzugt ausgewählt sind aus der Gruppe bestehend aus MMA, Methylmethacrylat; EMA, Ethylmethacrylat; n-BMA, n-Butylmethacrylat; IBMA, Isobutylmethacrylat, t-BMA, tert-Butylmethacrylat; EHMA, 2-Ethylhexylmethacrylat; LMA, Laurylmethacrylat; TDMA, Tridecylmethacrylat; SMA, Stearylmethacrylat; CHMA, Cyclohexylmethacrylat; BZMA, Benzylmethacrylat; IBXMA, Isobornylmethacrylate; MAA, Methacrylsäure; HEMA, 2-Hydroxyethylmethacrylat; HPMA, 2-Hydroxypropylmethacrylat; DMMA, Dimethylaminoethylmethacrylat; DEMA, Diethylaminoethylmethacrylat; GMA, Glycidylmethacrylat; THFMA, Tetrahydrofurfurylmethacrylat; AMA, Allylmethacrylat; ETMA, Ethoxyethylmethacrylat; 3FM, Trifluorethylmethacrylat; 8FM, Octafluorpentylmethacrylat; AIB, Isobutylacrylat; TBA, tert-Butylacrylat; LA, Laurylacrylat; CEA, Cetylacrylat; STA, Stearylacrylat; CHA, Cyclohexylacrylat; BZA, Benzylacrylat; IBXA, Isobornylacrylat; 2-MTA, 2-Methoxyethylacrylat; ETA, 2-Ethoxyethylacrylat; EETA, Ethoxyethoxyethylacrylat; PEA, 2-Phenoxyethylacrylat; THFA, Tetrahydrofurfurylacrylat; HEA, 2-Hydroxyethylacrylat; HPA, 2-Hydroxypropylacrylat; 4HBA, 4-Hydroxybutylacrylat; DMA, Dimethylaminoethylacrylat; 3F, Trifluorethylacrylat; 17F, Heptadecafluorodecylacrylat; 2-PEA, 2-Phenoxyethylacrylat; TBCH, 4-tert-butylcyclohexylacrylat; DCPA, Dihydrodicyclopentadienylacrylat; ; EHA, 2-Ethylhexylacrylat; und 3EGMA, Triethylenglycolmonomethacrylat.

11. Infiltrant nach einem der Ansprüche 1 bis 10, **dadurch gekennzeichnet, dass** er eine dynamische Viskosität von 50 mPas oder weniger, vorzugsweise 30 mPas oder weniger, weiter vorzugsweise 15 mPas oder weniger aufweist.

12. Infiltrant nach einem der Ansprüche 1 bis 11, **dadurch gekennzeichnet, dass** er 20 Gew.-% oder weniger, bevorzugt 10 Gew.-% oder weniger Lösungsmittel enthält.

13. Kit zur Herstellung eines Infiltranten nach einem der Ansprüche 1 bis 12, **dadurch gekennzeichnet, dass** der Kit eine erste Komponente mit Monomeren und chemisch aktivierbaren Initiatoren und eine zweite Komponente mit Aktivatoren enthält.

14. Kit zur Herstellung eines Infiltranten nach einem der Ansprüche 1 bis 12, **dadurch gekennzeichnet, dass** der Kit zusätzlich Ätz- und/oder Trocknungsmittel aufweist.

15. Infiltrant nach einem der Ansprüche 1 bis 14 zur Behandlung und/oder Prävention von kariösen Schmelzläsionen.

Europäisches
Patentamt

European
Patent Office

Office européen
des brevets

## EUROPÄISCHER RECHERCHENBERICHT

Nummer der Anmeldung

EP 08 01 1903

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 1 913 927 A (ERNST MUEHLBAUER GMBH & CO KG [DE]) 23. April 2008 (2008-04-23) * Seite 6, Absätze 32,36 * ----- | 1-5,7-15 | INV. A61K6/08 A61K6/083 |
| A | WO 2007/131725 A (CHARITE UNIVERSITAETSMEDIZIN [DE]; MEYER-LUECKEL HENDRIK [DE]; PARIS S) 22. November 2007 (2007-11-22) * Seite 1, Absatz 1 * * Seite 10, Absatz 4 * * Seite 13, Absätze 4,5 * ----- | 1-5,7-15 | |
| A | MEYER-LUECKEL ET AL: "Influence of the application time on the penetration of different dental adhesives and a fissure sealant into artificial subsurface lesions in bovine enamel" DENTAL MATERIALS, ELSEVIER, Bd. 22, Nr. 1, 1. Januar 2006 (2006-01-01), Seiten 22-28, XP005214018 ISSN: 0109-5641 * das ganze Dokument * ----- | | RECHERCHIERTE SACHGEBIETE (IPC) A61K A61L |

~~Der vorliegende Recherchenbericht wurde für alle Patentans~~prüche erstellt

| Recherchenort | Abschlußdatum der Recherche | Prüfer |
|---|---|---|
| Den Haag | 3. Dezember 2008 | Weiss, Marie-France |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Europäisches
Patentamt

European
Patent Office

Office européen
des brevets

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

7-15 (partiell), 1-5

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**Nummer der Anmeldung**

EP 08 01 1903

## MANGELNDE EINHEITLICHKEIT
## DER ERFINDUNG
## ERGÄNZUNGSBLATT B

Nach Auffassung der Recherchenabteilung entspricht die vorliegendeeuropäische Patentanmeldung nicht den Anforderungen an die Einheitlichkeit der Erfindungund enthält mehrere Erfindungen oder Gruppen von Erfindungen, nämlich:

1. Ansprüche: 1-5, 7-15 (partiell)

   Das Dentalmaterial weist einen Penetrationskoeffizienten von PK > 50 cm/s auf. Aufgrund des hohen PK-Wertes wird eine ausreichende Eindringtiefe des Harzes sichergestellt, sodass das Dentalmaterial eine gute Versiegelungswirkung aufweist.
   ---

2. Ansprüche: 6, 7-15 (partiell)

   Die vernetzenden Monomere mit mindestens drei polymerisierbaren Gruppen weisen einen Abstand der Vernetzungspunkte von wenigstens 7 Bindungslängen auf. Durch die Einstellung eines Abstandes zwischen den vernetzenden Gruppen werden in vernetzten Polymeren die Spannungen im ausgehärteten Polymer vermindert, sodass es nicht zu spannungsinduzierten Rissbildungen im Polymer kommt.
   ---

# EP 2 145 613 A1

| Im Recherchenbericht angeführtes Patentdokument | Datum der Veröffentlichung | Mitglied(er) der Patentfamilie | Datum der Veröffentlichung |
|---|---|---|---|
| EP 1913927 A | 23-04-2008 | KEINE | |
| WO 2007131725 A | 22-11-2007 | KEINE | |

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007131725 A1
- EP 1413569 A
- EP 1741419 A
- EP 1688125 A
- WO 2005086911 A
- WO 02066535 A
- WO 2005121200 A
- WO 02062901 A
- WO 2006031972 A
- EP 1714633 A
- EP 1285947 A
- EP 1849450 A
- US 2004017928 A1

**In der Beschreibung aufgeführte Nicht-Patentliteratur**

- **Fan P. L. et al.** Penetrativity of sealants. *J. Dent. Res.,* 1975, vol. 54, 262-264
- Appl. Sci. Publ. Elsevier, 1984, vol. 2